# EUROPEAN PATENT SPECIFICATION

(11) **EP 2 013 290 B1**
(45) Date of publication and mention of the grant of the patent: **12.06.2013**
(21) Application number: 07724242.8
(22) Date of filing: 13.04.2007
(51) Int. Cl.: C08L 89/00, C08J 5/18, C09D 189/00

(54) **MULTILAYER SILK PROTEIN FILMS**
MEHRSCHICHTIGE SEIDENPROTEINFILME
FILMS MULTICOUCHES DE PROTEINES DE SOIE

(30) Priority: 03.05.2006 EP 06113438
(43) Date of publication of application: 14.01.2009
(73) Proprietor: AMSilk GmbH, 82152 Planegg/Martinsried (DE)
(72) Inventor: SCHEIBEL, Thomas, 95448 Bayreuth (DE); SLOTTA, Ute, 95447 Bayreuth (DE)
(74) Representative: Zwicker, Jörk
(86) International application number: PCT/EP2007/003304
(87) International publication number: WO 2007/128378

(56) References cited:
- EP-A- 1 277 857
- US-A1- 2005 069 950
- D. HUEMMERICH; U. SLOTTA; T. SCHEIBER;: "Processing and modification of films made from recombinant spider silk proteins" APPLIED PHYSICS A, vol. 82, 22 November 2005 (2005-11-22), pages 219-222, XP008069585 cited in the application

## Description

The present invention is directed to a method of forming multilayer spider silk protein films and a multilayer film obtained therefrom. The invention is further directed to various materials, products and compositions containing said multilayer film and to the use of said multilayer film in several applications.

Multilayer films are useful for a large variety of purposes¹⁻¹². Applications could be said to fall into two general categories: tailoring interactions of a surface with its environment and fabricating "devices" with defined structural properties. The range of development areas includes coatings, colloid stabilization, light-emitting or photovoltaic devices, electrode modification, optical storage and magnetic films, high charge density batteries, biomaterials, alteration of biocompatibility, enzyme immobilization, flocculation for water treatment and paper making, functional membranes, separations, carriers, controlled release devices, sensors, and nanoreactors. A key attribute of the preferred method of preparing multilayer films and capsules is controlled vertical structuring on the nanometer scale. A polypeptide multilayer film is defined as a multilayer film made of polypeptides. In some instances another type of polymer is involved in the fabrication process, for instance a chemically modified polypeptide¹³, a non-biological organic polyelectrolyte¹⁴, or a polysaccharide¹⁵. A polypeptide film might be deposited to confer specific bio-functionality on a surface that was otherwise bio-inert or to convert a bioactive surface into one that is not adhesive to cells¹⁶⁻²⁰.

Multilayer films of polypeptides are promising for the development of applications which encompass some of the following desirable features: anti-fouling, biocompatibility, biodegradability, specific bio-molecular sensitivity, edibility, environmental benignity, thermal responsiveness, and stickiness or non-stickiness. Silk proteins are ideally suited for such applications by virtue of their biochemical nature, the control one can have over chemical structure in various approaches to polymer synthesis, the ability to control formation of secondary structure, or the availability of genomic data.

Patent application US 2005/0069950 A1 describes the fabrication of ultra thin multilayered films of polypeptides on suitable surfaces by electrostatic layer-by-layer self assembly (ELBL). Further, it describes a method for designing polypeptides for the nanofabrication of thin films for applications in biomedicine and other fields. A novel method for identifying sequence motifs of a defined length and net charge at neutral pH in amino acid sequence information for use in ELBL and recording of a desired number of the motifs is provided. US 2005/0069950 claims the use of small, highly charged peptides in ELBL assembly.

Spider silks are protein polymers that display extraordinary physical properties. Among the different types of spider silks, draglines are most intensely studied. Dragline silks are utilized by orb weaving spiders to build frame and radii of their nets and as lifelines that are permanently dragged behind. For these purposes high tensile strength and elasticity are required. The combination of such properties results in a toughness that is higher than that of most other known materials. Dragline silks are generally composed of two major proteins whose primary structures share a common repetitive architecture.

An orb web's capture spiral, in part composed of viscid silk formed by the flagelliform gland, which is therefore named flagelliform silk, is stretchy and can triple in length before breaking, but provides only half the tensile strength of dragline silk.

Systems for the recombinant production of spider silk proteins in *E. coli* have been developed earlier. As an example, it is referred to WO 2006/008163 A2, WO 2006/002827 A1, and US provisional application No. 60/712,095 (unpublished). In the expression system described therein, single building blocks (= modules) can be varied freely and can thus be adapted to the requirements of the specific case. Modules of this type are disclosed also in Ref. 21.

However, up to now, there is no general method of forming multilayer films from those silk proteins available. The methods which are known in the state of the art can not be used in a general way to produce various multilayer films made from silk proteins. As mentioned above, some publications rely on charged surfaces in order to stabilize the multilayer films, but do not provide a universal approach.

It is, therefore, an object of the present invention to provide a method for producing multilayer spider silk protein films and to provide films obtainable therefrom. It is a further object of the present invention to provide various applications for those multilayer films.

These objects are achieved by the subject-matter of the independent claims. Preferred embodiments are set forth in the dependent claims.

The inventors established for the first time a method for forming multilayer films made from silk proteins of different origin. They showed that multiple layers of silk proteins can be processed in order to form stable and useful multilayer films. This finding is unexpected since from the state of the art it could not be expected that stable multilayer films may be formed from silk proteins without creating specific conditions as, for example, oppositely charged layers.

According to a first aspect, the invention provides a method of forming multilayer spider silk protein films comprising the steps of:
a) providing one or more solutions of spider silk proteins dissolved or suspended in formic acid;
b) forming one of said solutions into a film;
c) evaporating the formic acid, thereby forming a first spider silk protein film layer; and
d) once or more repeating steps a) - c) to form additional spider silk protein film layers on said first spider silk protein film layer in order to form a multilayer spider silk protein film.

It has been known for a certain time to cast films made of spider silk proteins. Regarding the film casting of spider silk proteins see ref. 22. However, forming multilayer films was not described therein.

Apart from the fact that, surprisingly, multilayer films can be produced by the method of the present invention, the advantage is provided that tailored multilayer films can be produced. As an example, the thickness of the films may be controlled by the concentration of the employed protein solution. Furthermore, different silk proteins can be combined in the same or different layers in order to achieve the desired characteristics. Additionally, the present approach allows to combine the silk protein layers with other, for example, artificial polymers in order to achieve various purposes (as they will be outlined below in detail).

Additionally, it could surprisingly be shown that the single layers of spider silk protein films formed a stable multilayer film without the need for any modification of the surface of the single film layers (in contrast to the references cited above). This may be explained by the amphiphile character of the silk proteins which does not lead to a rejection of the different layers and, thus, allows for forming a stable multilayer film.

In an embodiment, each single layer of the multilayer film is formed from a spider silk protein solution comprising one or more types of spider silk proteins. Which types of silks may be used in practicing the present invention will be explained later. For example, the multilayer film is formed from layers comprising the same (homogenous multilayer film) or different (heterogenous multilayer film) spider silk proteins.

As mentioned above, in a preferred embodiment, the multilayer film comprises one or more layers made from spider silk proteins and one or more layers comprising other proteinaceous or non-proteinaceous materials.

In an embodiment, the thickness of one single layer of the multilayer film ranges from 0.5 to 1.5 µm. However, it is also possible to design thinner or thicker films (up to 3.0 µm). Further, a multilayer film of the invention preferably comprises 2-1000, preferably 2-100 and most preferably 2-20 layers.

The non-proteinaceous material is preferably selected from polystyrene, polyvinylchloride, poly(styrene sulfonate) (PSS), poly(allylamine hydrochloride) (PAH), poly(acrylic acid) (PAA), and/or poly(diallyldimethylammoniumchloride) (PDADMAC). The non-proteinaceous material may be used alone or in combination with other non-proteinaceous materials and/or silk proteins and/or other proteinaceous materials.

Those other proteinaceous materials may be preferably selected from collagens, elastin or keratin. Examples for those proteinaceous materials are mussel byssus proteins, for example those being obtained from *Mytilus sp.,* preferably from *M. edulis*, *M. galloprovincialis*, *M. californians*, or *Geukeria demissa.*

MASCOLO and WAITE (1986) first identified chemical gradients in byssus threads in *Mytilus.* After treatment of the threads with pepsin, two pepsin-resistant collagen fragments, called ColP and ColD, having molecular weights of 50 kDa and 60 kDa, respectively were identified. ColP can be found predominantly in the proximal area and is hardly to be found in the distal area. In contrast, the amount of ColD increases in the distal part to approximately 100% (LUCAS et al., 2002; QIN & WAITE, 1995). In the byssus thread as well as in the mussel foot, there is a further collagen-like protein which takes part in the construction of the thread structure. This additional protein is called ColNG (NG = no gradient), and is, in contrast to ColD and ColP, evenly distributed throughout the whole thread. Its physiological function presumably is being an adapter between the two other thread collagens (QIN & WAITE, 1998).

Keratins are a family of fibrous structural proteins which are tough and insoluble, form the hard but nonmineralized structures found in reptiles, birds and mammals. Keratins are also found in the gastrointestinal tracts of many animals, including roundworms. There are various types of keratins. Silk fibroins produced by insects and spiders are often classified as keratins.

Collagen is the main protein of connective tissue in animals and the most abundant protein in mammals, making up about 40% of the total. It is tough and inextensible, with great tensile strength, and is the main component of cartilage, ligaments and tendons, and the main protein component of bone and teeth. Collagen occurs in many places throughout the body, and occurs in different forms known as types, which include Type I to Type XIII collagen, among others (there are 27 types of collagen in total).

Elastin, is a protein in connective tissue that is elastic and allows many tissues in the body to resume their shape after stretching or contracting. Elastin helps skin to return to its original position when it is poked or pinched. It is primarily composed of the amino acids glycine, valine, alanine and proline. Elastin is made by linking many soluble tropoelastin protein molecules to make a massive insoluble, durable cross-linked array.

However, the present invention is not limited to these proteinaceous materials and many others may be used.

As a solvent, a polar solvent is used. The polar solvent is formic acid The silk proteins are solved or suspended in formic acid. Formic acid can easily be evaporated in order to leave behind the solved proteins, thus forming an individual film layer.

A "solution" in the meaning of the present invention means any liquid mixture that contains silk proteins and is amenable to film casting. Those solutions may also contain, in addition to protein monomers, higher order aggregates including, for example, dimers, trimers, and tetramers. The solutions may include additives to enhance preservation, stability, or workability of the solution.

A suspension herein is defined as a dispersion of solid particles in a liquid. If the particles are ∼100 nm in diameter, the suspension is colloidal.

In a preferred embodiment, the silk protein is selected from insect silk proteins or spider silk proteins, preferably natural or recombinant silk proteins, preferably silks from Insecta, Arachnida or analogues therefrom.

In particular preferred are the dragline and/or flagelliform sequences from dragline or flagelliform proteins of orb-web spiders (Araneidae and Araneoids).

Spider silks in general are protein polymers that display extraordinary physical properties, but there is only limited information on the composition of the various silks produced by different spiders (see Scheibel, 2004). Among the different types of spider silks, draglines from the golden orb weaver *Nephila clavipes* and the garden cross spider *Araneus diadematus* are most intensely studied. Dragline silks are generally composed of two major proteins and it remains unclear whether additional proteins play a significant role in silk assembly and the final silk structure. The two major protein components of draglines from *Araneus diadematus* are ADF-3 and ADF-4 (Araneus Diadematus Fibroin).

It is noted that the term "spider silk protein" as used herein does not only comprise all natural sequences but also all artificial or synthetic sequences which were derived therefrom.

Accordingly, the spider silk sequences may be derived from sequences which are termed "authentic" herein. This term means that the underlying nucleic acid sequences are isolated from their natural environment without performing substantial amendments in the sequence itself. The only modification, which is accepted to occur, is where the authentic nucleic acid sequence is modified in order to adapt said sequence to the expression in a host without changing the encoded amino acid sequence.

The authentic sequences are preferably derived from the amino terminal non-repetitive region (flagelliform proteins) and/or the carboxy terminal non-repetitive region (flagelliform and dragline proteins) of a naturally occuring spider silk protein. Preferred examples of those proteins will be indicated below.

According to a further embodiment, the authentic sequences are derived from the amino terminal non-repetitive region (flagelliform proteins) and/or the carboxy terminal non-repetitive region (flagelliform and dragline proteins) of a naturally occuring spider silk protein.

According to one preferred embodiment, the dragline protein is wild type ADF-3, ADF-4, MaSp I, MaSp II and the flagelliform protein is FLAG. The term ADF-3/-4 is used in the context of MaSp proteins produced by Araneus diadematus (Araneus diadematus fibroin-3/- 4). Both proteins, ADF-3 and -4 belong to the class of MaSp II proteins (major ampullate spidroin II). It is explicitely referred to WO2006/002827.

Monomeric sequence modules have been developed which are also forming a starting point of the present invention. These modules are derived from the genes ADF3 and ADF4 of the spider *Araneus diadematus* as well as the gene FLAG of the spider *Nephila clavipes.* Variations of the employed sequences of ADF3 and ADF4 are publicly available (available under the accession numbers U47855 and U47856). The first two genes (ADF3 and ADF4) are coding for proteins which are forming the dragline thread of the spider, the third is coding for a protein of the flagelliform silk. Based on the amino acid sequence of these proteins, several modules were designed:

| | | |
|---|---|---|
| Modul A: | GPYGPGASAA AAAAGGYGPG SGQQ | (SEQ ID NO: 1) |
| Modul C: | GSSAAAAAAA ASGPGGYGPE NQGPSGPGGY GPGGP | (SEQ ID NO: 3) |
| Modul Q: | GPGQQGPGQQ GPGQQGPGQQ | (SEQ ID NO: 2) |
| Modul K: | GPGGAGGPYG PGGAGGPYGP GGAGGPY | (SEQ ID NO: 4) |
| Modul sp: | GGTTIIEDLD ITIDGADGPI TISEELTI | (SEQ ID NO: 5) |
| Modul X: | GGAGGAGGAG GSGGAGGS | (SEQ ID NO: 6) |
| Modul Y: | GPGGAGPGGY GPGGSGPGGY GPGGSGPGGY | (SEQ ID NO: 7) |
| Modul A^{C}: | GPYGPGASAA AAAAGGYGPG CGQQ | (SEQ ID NO: 8) |
| Modul A^{K}: | GPYGPGASAA AAAAGGYGPG KGQQ | (SEQ ID NO: 9) |
| Modul C^{C}: | GSSAAAAAAA ASGPGGYGPE NQGPCGPGGY GPGGP | (SEQ ID NO: 10) |
| Modul C^{K1}: | GSSAAAAAAA ASGPGGYGPE NQGPKGPGGY GPGGP | (SEQ ID NO: 11) |
| Modul C^{K2}: | GSSAAAAAAA ASGPGGYGPK NQGPSGPGGY GPGGP | (SEQ ID NO: 12) |
| Modul C^{KC}: | GSSAAAAAAA ASGPGGYGPK NQGPCGPGGY GPGGP | (SEQ ID NO: 13) |
| Modul sp^{C}: | GGTTIIEDLD ITIDGADGPI TICEELTI | (SEQ ID NO: 14) |
| Modul sp^{K}: | GGTTIIEDLD ITIDGADGPI TIKEELTI | (SEQ ID NO: 15) |
| Modul X^{C}: | GGAGGAGGAG GCGGAGGS | (SEQ ID NO: 16) |
| Modul X^{K}: | GGAGGAGGAG GKGGAGGS | (SEQ ID NO: 17) |
| Modul Y^{C}: | GPGGAGPGGY GPGGSGPGGY GPGGCGPGGY | (SEQ ID NO: 18) |
| Modul Y^{K}: | GPGGAGPGGY GPGGSGPGGY GPGGKGPGGY | (SEQ ID NO: 19) |

From these amino acid modules, synthetic spider silk protein constructs were assembled. These modules and the spider silk proteins derived therefrom are among others forming the starting material in the present method of forming multilayer films.

The invention is further directed to the use of specific peptide TAGs. These tags (for example Tag's as disclosed in SEQ ID NO: 20-27, below) contain cysteine or lysine. The sequence of the TAG is so selected that an interaction with the rest of the silk protein and an influence of the assembling behavior can be precluded to the greatest possible extent.

The following Tag's were developed for preferred use in the spider silk constructs:

| | | |
|---|---|---|
| NH^{CYS1}: | GCGGGGGGSG GGG | (SEQ ID NO: 20) |
| NH^{CYS2}: | GCGGGGGG | (SEQ ID NO: 21) |
| NH^{LYS1}: | GKGGGGGGSG GGG | (SEQ ID NO: 22) |
| NH^{LYS2}: | GKGGGGGG | (SEQ ID NO: 23) |
| CH^{CYS1}: | GGGGSGGGGSGGCG | (SEQ ID NO: 24) |
| CH^{CYS2}: | GGGGSGGCG | (SEQ 10 NO: 25) |
| CH^{LYS1}: | GGGGSGGGGS GGKG | (SEQ ID NO: 26) |
| CH^{LYS2}: | GGGGSGGKG | (SEQ ID NO: 27) |

Preferred examples of synthetic silk proteins made from these modules may be found in chapter examples and preferably are (AQ)₂₄NR3 and C₁₆.

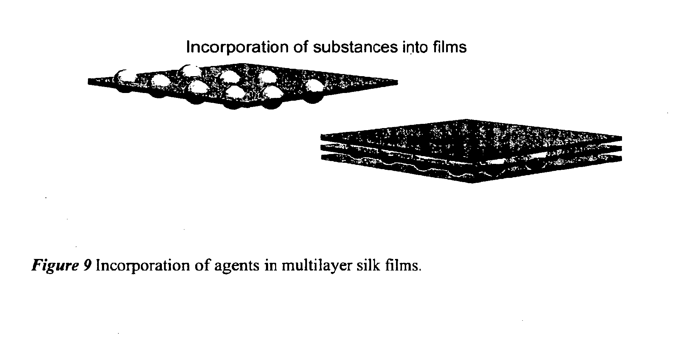

The layers of the multilayer film preferably comprise one or more agents incorporated therein or located between two adjacent layers. Those agents preferably are selected from salts, dyes, metals, chemicals and/or pharmaceutical agents. In this regard, it is referred to Figures 9, 11 and 12 which show the principles of such an incorporation. The substances to be incorporated may be solid, semi-solid or liquid without limitation.

For example, the pharmaceutical agent may be selected from the group consisting of analgetics; hypnotics and sedatives; drugs for the treatment of psychiatric disorders such as depression and schizophrenia; anti-epileptics and anticonvulsants; drugs for the treatment of Parkinson's and Huntington's disease, aging and Alzheimer's disease; drugs aimed at the treatment of CNS trauma or stroke; drugs for the treatment of addiction and drug abuse; chemotherapeutic agents for parasitic infections and diseases caused by microbes; immunosuppressive agents and anti-cancer drugs; hormones and hormone antagonists; antagonists for non-metallic toxic agents; cytostatic agents for the treatment of cancer; diagnostic substances for use in medicine; immunoactive and immunoreactive agents; antibiotics; antispasmodics; antihistamines; antinauseants; relaxants; stimulants; cerebral dilators; psychotropics; vascular dilators and constrictors; anti-hypertensives; drugs for migraine treatment; hypnotics, hyperglycemic and hypoglycemic agents; anti-asthmatics; antiviral agents; and mixtures thereof.

Further, multilayer films according to the invention may be designed which are acting as drug delivery system topically or systemically. A topical system may comprise a multilayer system, wherein a viscous liquid is incorporated and which system is applied to the skin for a predefined time. During that time, the liquid penetrates through one or more layers of said multilayer film, thus providing a defined amount of said liquid to the skin surface. In this case, the multilayer film may be regarded as TTS (transdermal therapeutic system). Pharmaceutical substances as hormones or nicotine might be administered by that way.

As an alternative, solid substances might be incorporated within and/or between one or more layers of said multilayer. After oral administration, the particles will migrate through the layers or will be forced out by influx of body fluids (omotic systems) or by slowly dissolving one or more of the outer layers and subsequent (sustained) release of the respective substance.

In other applications, for example clothing, the multilayer film may be specifically adapted to the required field of use, for example, might have one or more windtight or waterresistant layers. Additionally, as an example, silver might be incorporated into the layers in order to provide an antiseptic effect.

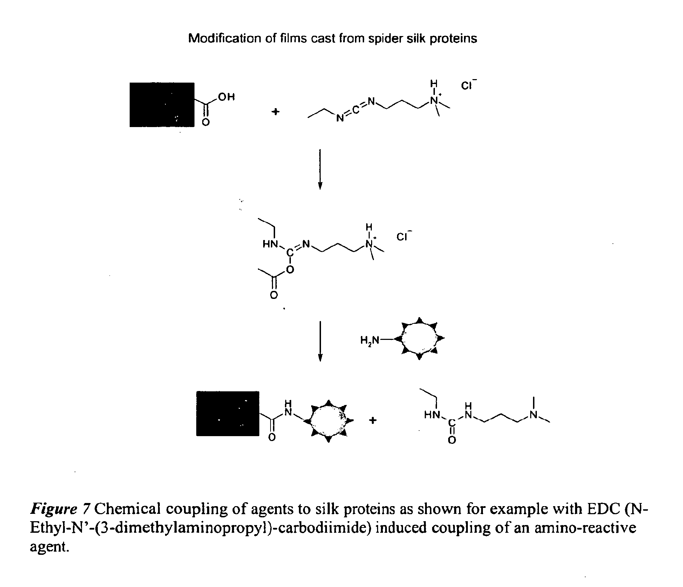

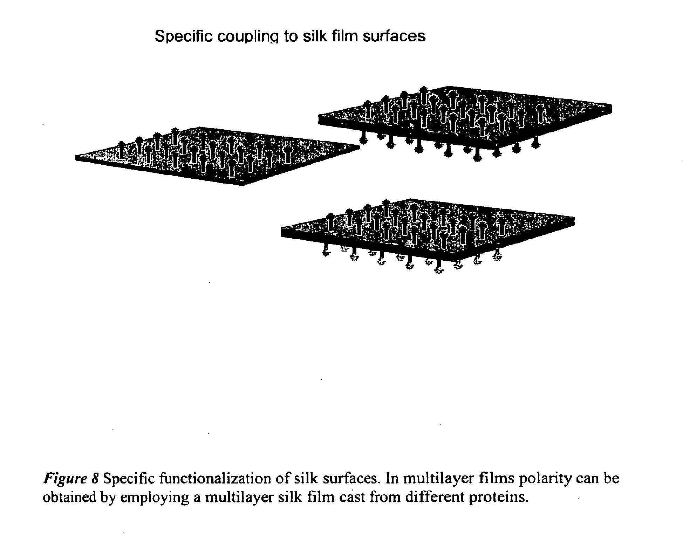

According to a further preferred embodiment, the silk proteins are covalently functionalized before or after step 1b) as defined above. In this connection, it is also referred to the accompanying examples, see Figures 7 and 8.

The layers may also be further processed in order to achieve additional characteristics. For example, since water insolubility is a prerequisite for most applications of protein films (which are mostly water soluble), the films may be processed in order to become water-insoluble. Suitable methods for this purpose are treatment with potassium phosphate or methanol.

The spider silk protein solution for casting the respective layer of the multilayer film is containing 0.1-20%, preferably 0.5-10%, more preferably 1-3% w/v of spider silk protein. It is important to note that the concentration of the solution is crucial since it will determine the actual thickness of the film. Also by this means, tailored multilayer films of layers having a predetermined thickness can be produced adapted to the specific envisioned application.

The layers of the multilayer film of the present invention may be formed by any available method, preferably by moulding, spincoating or casting the solution onto a suitable support. The type of support is generally not restricted, however, supports of polystyrene, glass or silane (or any other surface which is resistant to the employed solvents) may be named as suitable supports.

In a second aspect, the present invention is directed to a multilayer film obtainable by the method as defined above. A multilayer spider silk protein film of the invention comprises at least two layers of spider silk protein films.

According to a third aspect, the invention provides a cosmetical composition; a pharmaceutical or medical composition, preferably drug delivery system, artificial cell, contact lens coating, sustained-release drug delivery system, artificial skin graft; food composition; automotive part; aeronautic component; computer or data storage device; building material; textile or membrane comprising the above multilayer film.

Furthermore, the present invention provides the use of that multilayer film in medicine.

The present invention will be illustrated by the following non-limiting Examples and the accompanying figures.

The figures are showing the following:
***Figure 1*** CD-spectra of synthetic silk proteins (AQ)₂₄NR3 and C₁₆ dissolved in 6 M guanidinium thiocyanate followed by dialysis against 5 mM potassium phosphate pH 8.0 (straight line) or dissolved in HFIP (dotted line).
***Figure 2*** CD-spectra of protein films made from (AQ)₂₄NR3 and C₁₆. Films were cast from a protein solution in HFIP directly on a plain quartz glass and analyzed by CD-spectroscopy (dotted line). The film was subsequently processed with 1 M potassium phosphate and re-analyzed. Due to inaccuracies in defining the thickness of the films, Θ_{MRW} could not be determined.
***Figure 3*** Modification of C₁₆ films cast from a HFIP solution and processed with potassium phosphate. (A) Efficient coupling of fluorescein (yellow colour) only occurred when the carboxyl groups of C₁₆ were activated (+) using EDC. In contrast only little fluorescein bound to films without EDC activation (-). (B) Activity of coupled β-galactosidase was monitored using X-Gal as substrate. The occurrence of a blue precipitate indicated enzyme activity only on films that had been activated with EDC (+), while non-activated films only showed residual enzymatic activity (-).
***Figure 4*** Casting of multilayer silk films.
***Figure 5*** Casting of multilayer silk films with different functionalities.
***Figure 6*** Casting of multilayer silk films with different functionalities.
***Figure 7*** Chemical coupling of agents to silk proteins as shown for example with EDC (N-Ethyl-N'-(3-dimethylaminopropyl)-carbodiimide) induced coupling of an amino-reactive agent.
***Figure 8*** Specific functionalization of silk surfaces. In multilayer films polarity can be obtained by employing a multilayer silk film cast from different proteins.
***Figure 9*** Incorporation of agents in multilayer silk films.
***Figure 10*** Incorporation of agents in multilayer silk films. Differently colored chemicals were added to the silk protein solution prior to casting as a proof of principle.
***Figure 11*** Incorporation of solid agents in a sandwich multilayer silk film.
***Figure 12*** Incorporation of fluid agents in a sandwich multilayer silk film.

### Examples:

### Film casting of spider silk proteins:

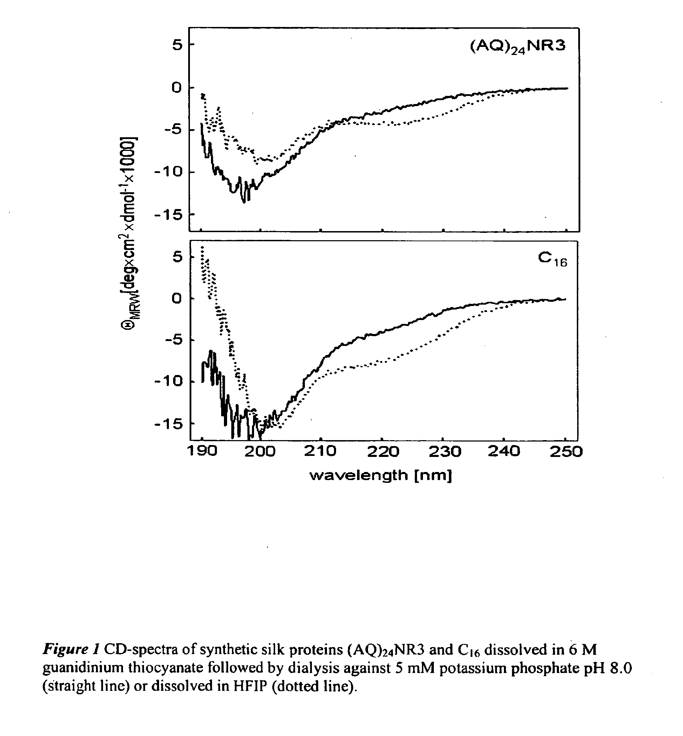

In order to cast films the inventors previously employed the two synthetic silk proteins, (AQ)₂₄NR3 and C₁₆, which are derived from the dragline silk proteins ADF-3 and ADF-4 from the garden spider *Araneus diadematus.* These proteins were chosen based on previous observations that ADF-3 and ADF-4 as well as its derivatives display a markedly different solubility and assembly behaviour. Measuring circular dichroism (CD) of (AQ)₂₄NR3 and C₁₆ solutions revealed a different influence of aqueous buffer and HFIP on secondary structure. In aqueous solution both proteins displayed a CD-spectrum with a single minimum at a wavelength below 200 nm which is indicative of a mainly random coiled protein (Figure 1).

In contrast, the spectra of both proteins in HFIP displayed one minimum at 201 - 202 nm and an additional minimum ((AQ)₂₄NR3) or shoulder (C₁₆) at 220 nm which is indicative of an increased α-helical content (Figure 1). Such an effect of fluorinated alcohols on proteins and peptides has been reported previously and has also been observed for silk fibroin and a synthetic silk protein derived from the dragline silk protein MaSpl from the spider *Nephila clavipes.*

Films were cast from 200 µl HFIP solutions containing 2% w/v protein on a polystyrene surface (or on quartz glass for CD-measurements). After evaporation of the solvent, (AQ)₂₄NR3 and C₁₆ both formed transparent films that could easily be peeled off the surface. Assuming complete evaporation of the solvent and the density of the protein film to be identical with the reported value of 1.3 g/cm³ for spider dragline silk, the thickness of the films was calculated to range from 0.5 to 1.5 µm. As-cast (freshly prepared) films made of either protein dissolved upon contact with water. Since water insolubility is a prerequisite for most applications of protein films, we searched for a processing method in order to render films insoluble. Potassium phosphate is known to induce aggregation and formation of chemically stable structures of the employed silk proteins. Also methanol has been used to obtain insoluble silk morphologies. Accordingly, processing (incubating) of as-cast films with 1 M potassium phosphate or methanol resulted in the conversion of water-soluble films into water-insoluble ones.

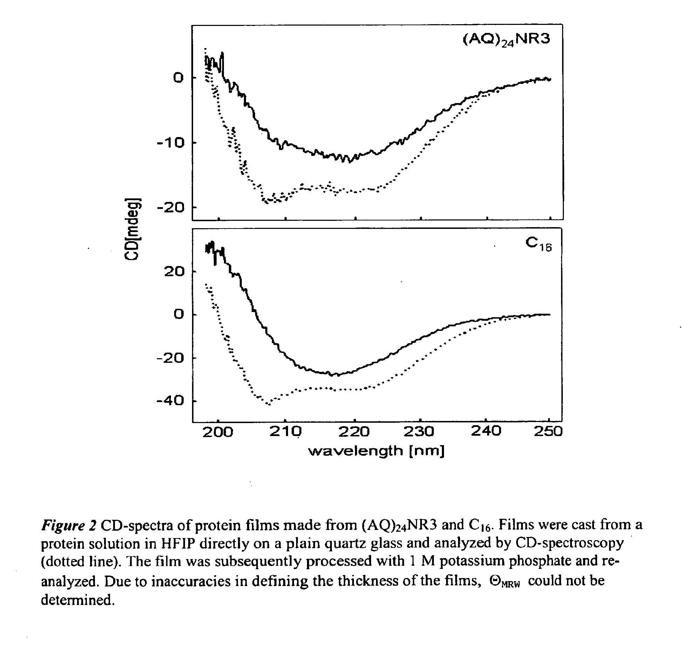

To investigate the structural properties of the films, the secondary structure of the underlying proteins was investigated by CD-spectroscopy. As-cast films revealed a spectrum with two pronounced minima at 208 nm and 220 nm indicative of an α-helical content higher than that of soluble proteins. After processing with 1 M potassium phosphate, films revealed spectra with a single minimum at 218 nm, which is typical for a β-sheet rich protein structure (Figure 2). Similar results were obtained after processing films with methanol (data not shown). Thus, the transition from water-solubility to water-insolubility was paralleled by a conversion of the protein's secondary structure from α-helix to β-sheet.

To test their chemical stability, films were submerged for 24 hours in 8 M urea, 6 M guanidinium hydrochloride and 6 M guanidinium thiocyanate. As-cast films of both proteins as well as (AQ)₂₄NR3 films processed with potassium phosphate or methanol were soluble in all of these denaturants. In contrast, C₁₆ films processed with potassium phosphate or methanol could only be dissolved in guanidinium thiocyanate. This remarkable chemical stability of C₁₆ films is identical to that of natural dragline silk and to that of recombinantly produced and assembled ADF-4. Previous studies could correlate assembly properties and stabilities of assembled structures directly with the amino acid sequences of the silk proteins. Thus, properties of spider silk films can directly be modified by altering the primary structure of the silk protein via manipulation of the corresponding silk gene (Figure 2).

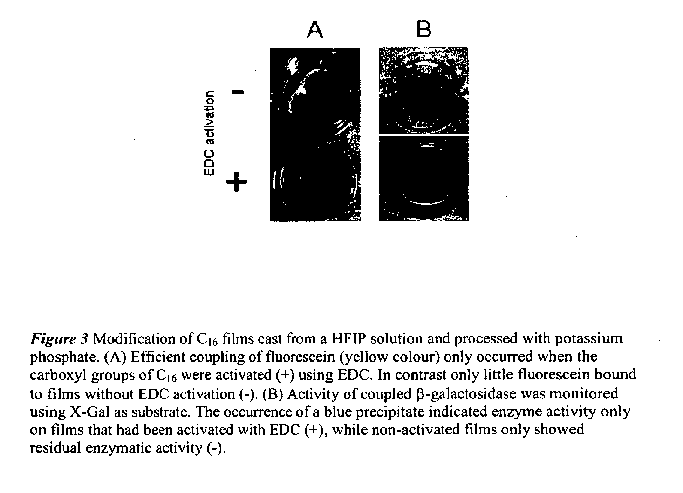

Many applications of protein films require the presence of specific functionalities on the film's surface. In order to demonstrate, that the employed spider silk films can be modified with small organic molecules as well as biological macromolecules such as proteins, the chromophor fluorescein and the enzyme β-galactosidase were chemically coupled to C₁₆ films processed with potassium phosphate as a proof of principle. The coupling was achieved by activating surface exposed carboxyl groups of C₁₆ using 1-ethyl-3-(3-dimethylaminopropyl)carbodiimide (EDC). The films were further incubated with ethylenediamine leading to the formation of an amide. The remaining free amino group of ethylenediamine was subsequently coupled to fluoresceinisothiocyanate resulting in the efficient covalent linkage of fluorescein via formation of a stable thiourea derivative (Figure 3). Similarly, incubation of β-galactosidase with EDC-activated C₁₆ films led to the fonnation of amide bonds between carboxyl groups of C₁₆ and primary amines (e.g. from lysine residues) of β-galactosidase which were accessible at the enzyme's surface. After repeated washing of such modified films, β-galactosidase activity could be detected using 5-bromo-4-chloro-3-indolyl-β-D-galactopyranoside (X-Gal) as a substrate (Figure 3).

The inventors could demonstrate that protein films can be obtained from synthetic spider silk proteins. The films, which initially were water soluble, can be processed with potassium phosphate or methanol leading to water-insolubility, which is a major requirement for many applications. Comparison of the chemical stabilities of films made from two different synthetic spider silk proteins suggested that the properties of the films were based on the primary structure of the proteins. Employing our previously established cloning strategy for spider silk genes, it will be possible to generate silk proteins that form films displaying specific properties. Since different functional molecules can be covalently attached to the film's surface, a great variety of technical or medical applications can therefore be approached.

The proteins (AQ)₂₄NR3 and C₁₆ can be cast into several films starting from HFIP or formic acid solutions. However, any other silk protein built upon our modules (sequence 1-27) as well as natural insect and spider silk proteins can be employed. The protein solution is cast on polystyrene, glass or silane (or any other surface which are resistant to the employed solvents) and the solvent is completely evaporated afterwards. Films cast from hexafluoroisopropanol solutions are water soluble. To achieve water-insolubility these films have to be treated with methanol, ethanol or potassium phosphate. Films cast from formic acid are insoluble in water without processing.

The thickness of the films can be controlled by the concentration of the employed protein solution (data not shown). Importantly, films can be cast from solutions of a single protein (One-protein films) or of two proteins (Two-protein films).

### Two-protein films

The inventors analyzed whether films cast from solutions containing two protein components (Two-protein films) revealed a different structure or stability in comparison to One-protein films. (AQ)₁₂ and C₁₆ or (AQ)₂₄NR3 and C₁₆NR4 were dissolved in HFIP (1% w/v each, 2% w/v in total). For sequence information, it is referred to WO2006/008163, incorporated herein by reference.

Two-protein films were cast from (AQ)₁₂/C₁₆ (molar ratio 1 : 1) or (AQ)₂₄NR3/C₁₆NR4 (molar ratio 1 : 1.8) mixtures. Remarkably, Two-protein films showed a combination of the properties of the films cast from the single silk proteins. As-cast Two-protein films made of (AQ)₁₂/C₁₆ have been soluble in all tested reagents. After processing with methanol, these films became insoluble in water and urea, but soluble in solutions of GdmCl and GdmSCN, reflecting a chemical stability between that of plain C₁₆ or plain (AQ)₁₂ films. As-cast Two-protein films of (AQ)₂₄NR3/C₁₆NR4 could not be completely dissolved in water. After water treatment, amorphous protein aggregates were remaining. The formation of intermolecular disulphide bridges between the NR-regions could be excluded to cause amorphous aggregation, since the behaviour of the films did not change in the presence of a reducing agent such as β-mercaptoethanol (5% (v/v)). Processing of the (AQ)₂₄NR3/C₁₆NR4 films with methanol led to chemical stability in water and urea.

In the case of Two-protein films, the treatment with methanol led to a chemical stability influenced by both proteins. Compared to conventional synthetic polymers, where blending usually causes mixed properties, such finding is astonishing for proteins. In general, the structure and interaction of proteins is complex and depends on many factors. When mixed, two proteins, that do not interact, usually remain their chemical stability found in the absence of the second protein. In case they do interact, usually both proteins show a higher chemical stability. In our case, both proteins seemed to interact, but the gained chemical stability was between that of the single compounds.

### Multilayer films

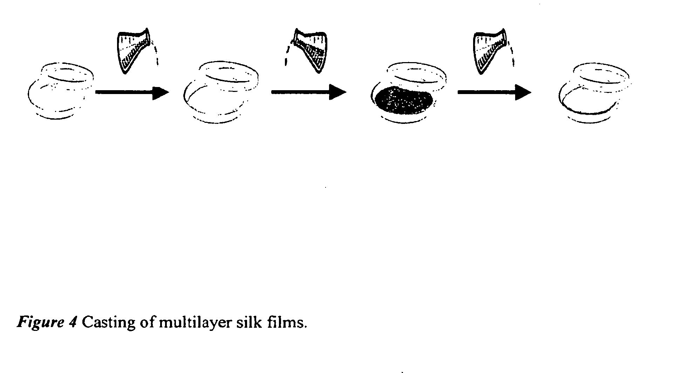

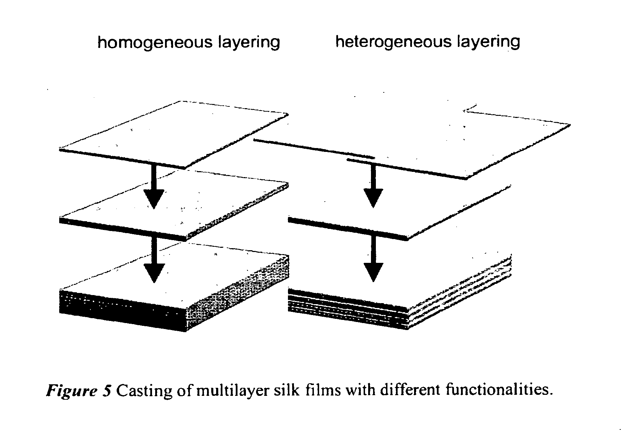

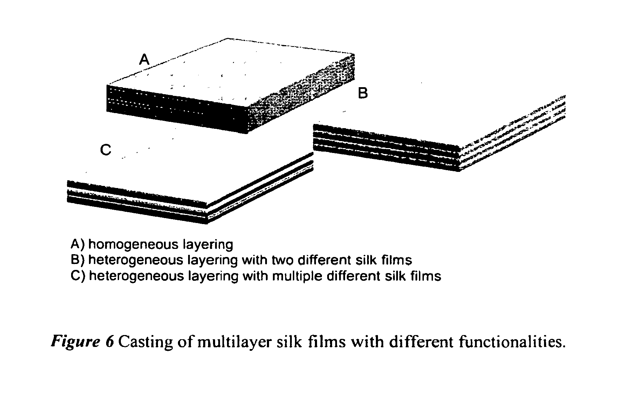

Multilayered films can be obtained by casting further layers on already existing films (Figure 4, 5, and 6). All layers of a multilayer film can be made of spider silk, but film layers can additionally be made of other materials such as insect silk, elastin, collagen, keratin, polystyrene, polyvinylchloride, poly(styrene sulfonate) (PSS), poly(allylamine hydrochloride) (PAH), poly(acrylic acid) (PAA), poly(diallyldimethylammoniumchloride) (PDADMAC), etc. The thickness of the silk films can be controlled by the protein concentration. Each layer can contain a different silk protein (natural insect or spider silk, or recombinant silk based on our modules sequence 1-27) with different chemical and physical properties. Further, each film can contain differently modified silk proteins (the modification can take place before film casting). Finally, each layer can be post-cast processed with a desired functionality by chemically coupling an agent to the respective silk protein (Figures 7 and 8).

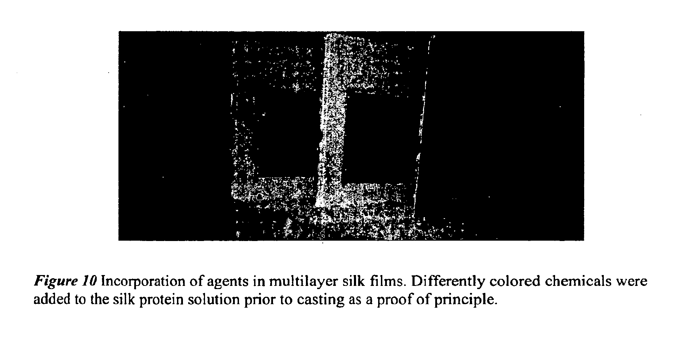

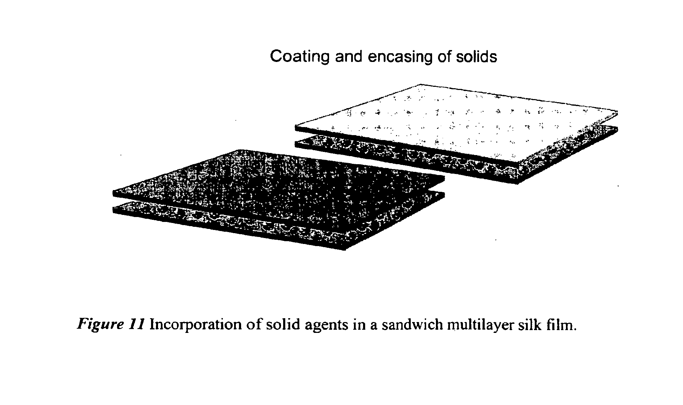

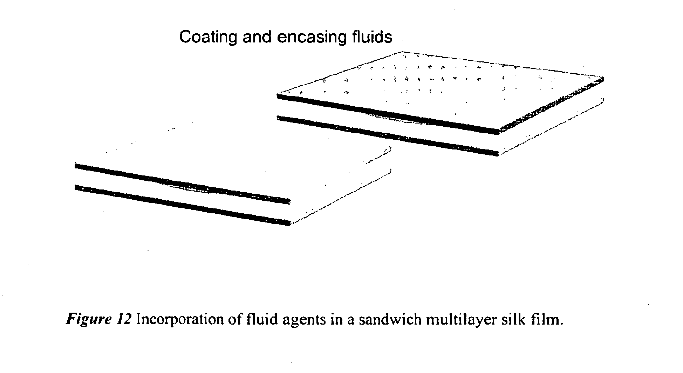

Substances can be incorporated into films cast from recombinant spider silk proteins by adding them before casting (Figure 9 and 10). Alternatively the substance can be given on top of a silk film and another silk film can be cast on it (Figures 11 and 12).

**As a summary, the following objects can be achieved by the present invention:**
Single spider silk film layers can either be covalently functionalized before or after casting. Further, blends between spider silk and agents (such as salts, dyes, metals, chemicals, drugs, etc.) can be prepared prior to casting. Casting layer by layer generates multilayer films with different functionalities in each layer. Such multilayer multifunctional spider silk protein films are entirely new. Additionally, blending of different spider silk proteins, each functionalized differently, for casting a single film is new. Thereby, each single film of a multilayer protein film can provide different functionalities, yielding a complex three-dimensional scaffold with defined spatial distributions of single functions. In case, the single function can communicate, smart three-dimensional structures are the result. Finally, silk films can be layered with other existing polymer films, creating mixed multilayer films of different components.

### Industrial applicability of the invention:

Multilayer, multifunctional scaffolds and structures are a basis for a huge amount of innovative products for food science, waste disposal, and the cosmetical, medical, pharmaceutical, automotive, aeronautic, etc. market. The applications involve for example, device coatings (e.g. for advanced endothelial cell attachments), drug delivery systems, artificial cells, contact lens coatings, sustained-release drug delivery systems, biosensors, and functionally advanced materials with various electrical (e.g. light-emitting diodes), magnetic, electrochromic, and optical properties (e.g. enhancing the brightness of handheld computers and computer screens, reducing the amount of interference with cellular signals, and enabling automobiles to function more efficiently by reflecting infrared light (heat rays) and thereby reducing the burden on air conditioners).

Multilayer spider silk films can take light that would normally be absorbed and turn it into useful light, and increase brightness. In automotive industry multilayer silk films can be employed that reflect solar infrared heat, since the non-conductive films are completely clear, a property that could be useful in architectural applications where increased daylight transmission is desirable. Multilayer film that are comprised of hundreds of layers of transparent silk polymers reflect due to optical interference effects. The wavelengths that are reflected and transmitted change as a result of the angle at which they're held.

Multilayers films can been used to form thin nanoporous and microporous membranes and can been exploited as nanoreactors for the synthesis of metallic nanoparticles.

The hydrogen-bonded multilayer spider silk films can be employed in applications such as micropatteming and drug delivery, where control of the deconstruction rate of the hydrogen-bonded films is desirable. One approach to modulating the deconstruction behavior of multilayered thin films is via structural design of the films.

Another application would be enantiomeric separations. Chirality is key to molecular recognition in biology. The pharmaceutical industry's need for single enantiomer drugs has spurred the development of techniques for preparative-scale separation of chiral molecules. Membrane-based separations have attracted much attention due to operational simplicity and low cost.

Further applications of multilayer spider silk films are artificial skin grafts. Major burn accidents involve extensive damage to the skin. Immediate coverage is needed to limit loss of fluid and aid tissue repair and regeneration. The structural and functional properties of an ideal skin substitute should closely match autograft skin. Plasticity of the substitute preparation procedure and its composition provide added value for coverage, minimizing rejection and activation of the inflammatory response. Multilayer spider silk films could be useful for preparing artificial skin grafts, as the method provides a simple means of producing films without limit to size, shape, and composition and polypeptides are inherently biocompatible.

Nanofiltration (NF) is a pressure-driven membrane separation process that is used for applications such as water softening, brackish water reclamation, and dyesalt separations. Such applications do not require the high NaCl rejections that are typical of reverse osmosis (RO) membranes, so NF occurs at significantly lower pressures than RO and, hence, requires less energy. For this reason, the use of NF is growing rapidly, but continued development of selective, high-flux membranes that are stable and resist fouling should enhance the utility of this separation technique. In both RO and NF, membranes consist of a selective skin layer on a highly permeable support because the minimal thickness of the skin layer allows a reasonable flux despite its dense nature. Typical procedures for creating such membrane structures include phase inversion and formation of composite membranes by interfacial polymerization, grafting, or film deposition on a preformed porous support. Composite membranes are particularly attractive because they require only small amounts of the potentially expensive skin material. Multilayer spider silk films provide a controlled method for forming the skin layer of membranes for NF, gas-separation, and pervaporation.

### Literature

(1) Decher, G. Science 1997, 277, 1232.
(2) Chen, W.; McCarthy, T. J. Macromolecules 1997, 30, 78.
(3) Bertrand, P.; Jonas, A.; Laschewsky, A.; Legras, R. Macromol. Rapid Comm. 2000, 21, 319.
(4) Hammond, P. T. Curr. Opin. Colloid Interface Sci. 2000, 4, 430.
(5) Anzai, J.-I. Bunseki Kagaku 2001, 50, 585.
(6) Tripathy, S. K., Kumar, J., Nalwa, H. S., Eds.; Handbook of Polyelectrolytes and their Applications. Vol. 1. Polyelectrolyte-based Multilayers, Self-assemblies and Nanostructures; American Scientific Publishers: Stevenson Ranch, CA, 2002.
(7) Decher, G., Schlenoff, J. B., Eds.; Multilayer Thin Films: Sequential Assembly of Nanocomposite Materials; Wiley-VCH: Weinheim, Germany, 2003.
(8) Schonhoff, M. Curr. Opin. Colloid Interface Sci. 2003, 8, 86.
(9) Schonhoff, M. J. Phys.-Condens. Mater. 2003, 15, R1781.
(10) Hammond, P. T. Ad. Mater. 2004, 16, 1271.
(11) Muller, M.; et al.. Adhesion 2004, 80, 521.
(12) Peyratout, C. S.; Da¨hne, L. Angew. Chem., Int. Ed. 2004, 43, 3762.
(13) Boulmedais, F.; et al. Biomaterials 2004, 25, 2003.
(14) Hubsch, E.; et al. Langmuir 2005, 21, 3664.
(15) Picart, C.; et al. Proc. Natl Acad. Sci. U.S.A. 2002, 99, 12531.
(16) Elbert, D. L.; Herbert, C. B.; Hubbell, J. A. Langmuir 1999, 15, 5355.
(17) Richert, L.; et al. Biomacromolecules 2002, 3, 1170.
(18) Halthur, T. J.; Claesson, P. M.; Elofsson, U. M. J. Am. Chem. Soc. 2004, 126, 17009.
(19) Richert, L.; Arntz, Y.; Schaaf, P.; Voegel, J.-C.; Picart, C. Surf. Sci. 2004, 570, 13.
(20) Haynie, D.; et al. Biomacromolecules 2005, 6, 2895.
(21) Hümmerich, D.; Helsen, C.W.; Oschmann, J.; Rudolph, R.; Scheibel, T. Biochemistry 2004, 43, 13604.
(22) Hümmerich, D.; Slotta, U.; Scheibel, T. Applied Physics A 2006, 82, 219.

## Claims

1. A method of forming multilayer spider silk protein films comprising the steps of:
a) providing one or more solutions of spider silk proteins dissolved or suspended in formic acid;
b) forming one of said solutions into a film;
c) evaporating the formic acid, thereby forming a first spider silk protein film layer; and
d) once or more repeating steps a) - c) to form additional spider silk protein film layers on said first spider silk protein film layer in order to form a multilayer spider silk protein film.

2. The method of claim 1, wherein each single layer of the multilayer film is formed from a spider silk protein solution comprising one or more types of spider silk protein.

3. The method of one or more of the preceding claims, wherein the multilayer film is formed from layers comprising the same (homogenous multilayer film) or different (heterogenous multilayer film) spider silk proteins.

4. The method of one or more of the preceding claims, wherein the multilayer film comprises one or more layers made from spider silk proteins and one or more layers comprising other proteinaceous or non-proteinaceous materials, wherein :
the non-proteinaceous material preferably is selected from polystyrene, polyvinylchloride, poly(styrene sulfonate) (PSS), poly(allylamine hydrochloride) (PAH), poly(acrylic acid) (PAA), and/or poly(diallyldimethyl-ammoniumchloride) (PDADMAC), and/or wherein the other proteinaceous material preferably is selected from collagens, elastin or keratin.

5. The method of one or more of the preceding claims, wherein the spider silk proteins are natural or recombinant silk proteins, preferably silks from Arachnida or analogues therefrom.

6. The method of one or more of the preceding claims, wherein the layers of the multilayer film comprises one or more agents incorporated therein or located between two adjacent layers, wherein the one or more agents preferably are selected from metals, chemicals and/or pharmaceutical agents.

7. The method of one or more of the preceding claims, wherein the spider silk proteins are covalently functionalized before or after step 1b).

8. The method of one or more of the preceding claims, wherein the spider silk protein solution is containing 0.1-20%, preferably 0.5-10%, more preferably 1-3% w/v of spider silk protein.

9. The method of one or more of the preceding claims, wherein the film is formed by moulding, spincoating or casting the solution onto a suitable support.

10. A multilayer film obtainable by the method of one or more of claims 1-9.

11. A multilayer spider silk protein film comprising at least two layers of spider silk protein films, wherein said films are cast from formic acid.

12. The multilayer film of claim 11, wherein each single layer of the multilayer film is formed from a spider silk protein solution comprising one or more types of spider silk protein.

13. The multilayer film of claims 11 or 12, wherein the multilayer film is formed from layers comprising the same (homogenous multilayer film) or different (heterogeneous multilayer film) spider silk proteins, and/or, wherein the multilayer film comprises one or more layers made from spider silk proteins and one or more layers comprising other proteinaceous or non-proteinaceous materials, wherein the non-proteinaceous material preferably is selected from polystyrene, polyvinylchloride, poly(styrene sulfonate) (PSS), poly(allylamine hydrochloride) (PAH), poly(acrylic acid) (PAA), and/or poly(diallyldimethyl-ammoniumchloride) (PDADMAC), and/or wherein the other proteinaceous material preferably is selected from collagens, elastin or keratin.

14. The multilayer film of one or more of claims 11-13, wherein the spider silk proteins are natural or recombinant silk proteins, preferably silks from Arachnida or analogues therefrom.

15. The multilayer film of one or more of claims 11-14, wherein the layers of the multilayer film comprise one or more agents incorporated therein or located between two adjacent layers wherein the one or more agents are preferably selected from metals, chemicals and/or pharmaceutical agents, and/or wherein the spider silk proteins are covalently functionalized.

## Patentansprüche

1. Verfahren zur Bildung von mehrschichtigen Spinnenseidenproteinfilmen, welches die Schritte umfasst:
a) Bereitstellen von einer oder mehreren Lösungen von Spinnenseidenproteinen, die in Ameisensäure gelöst oder suspendiert sind;
b) Bilden eines Films aus einer dieser Lösungen;
c) Evaporieren der Ameisensäure, wobei eine erste Spinnenseidenproteinfilmschicht gebildet wird; und
d) einmaliges oder mehrmaliges Wiederholen der Schritte a) - c), um zusätzliche Spinnenseidenproteinfilmschichten auf der ersten Spinnenseidenproteinfilmschicht zu formen, um einen mehrschichtigen Spinnenseidenproteinfilm zu bilden.

2. Verfahren nach Patentanspruch 1, wobei jede einzelne Schicht des mehrschichtigen Films aus einer Spinnenseidenproteinlösung gebildet wird, die eine oder mehrere Arten von Spinnenseidenprotein umfasst.

3. Verfahren nach einem oder mehreren der vorangegangenen Patentansprüche, wobei der mehrschichtige Film von Schichten, die die gleichen (homogener mehrschichtiger Film) oder verschiedene (heterogener mehrschichtiger Film) Spinnenseidenproteine umfassen, gebildet wird.

4. Verfahren nach einem oder mehreren der vorangegangenen Patentansprüche, wobei der mehrschichtige Film eine oder mehrere aus Spinnenseidenproteinen hergestellte Schichten und eine oder mehrere Schichten, die andere proteinartige oder nicht-proteinartige Materialien umfassen, umfasst, wobei
das nicht-proteinartige Material vorzugsweise aus Polystyrol, Polyvinylchlorid, Poly(styrolsulfonat) (PSS), Poly(allylaminhydrochlorid) (PAH), Poly(acrylsäure) (PAA) und/oder Poly(diallyldimethylammoniumchlorid) (PDADMAC) ausgewählt ist und/oder wobei das andere proteinartige Material vorzugsweise aus Kollagenen, Elastin oder Keratin ausgewählt ist.

5. Verfahren nach einem oder mehreren der vorangegangenen Patentansprüche, wobei die Spinnenseidenproteine natürliche oder rekombinante Seidenproteine sind, vorzugsweise Seiden von Arachnida oder Analoga davon.

6. Verfahren nach einem oder mehreren der vorangegangenen Patentansprüche, wobei die Schichten des mehrschichtigen Films einen oder mehrere Wirkstoffe umfassen, die darin inkorporiert sind oder sich zwischen zwei benachbarten Schichten befinden, wobei der eine oder die mehreren Wirkstoffe vorzugsweise aus Metallen, Chemikalien und/oder pharmazeutischen Wirkstoffen ausgewählt sind.

7. Verfahren nach einem oder mehreren der vorangegangenen Patentansprüche, wobei die Spinnenseidenproteine vor oder nach Schritt 1 b) kovalent funktionalisiert werden.

8. Verfahren nach einem oder mehreren der vorangegangenen Patentansprüche, wobei die Spinnenseidenproteinlösung 0,1-20%, vorzugsweise 0,5-10%, bevorzugter 1-3% w/v Spinnenseidenprotein umfasst.

9. Verfahren nach einem oder mehreren der vorangegangenen Patentansprüche, wobei der Film durch Formen, Rotationsbeschichten oder Gießen der Lösung auf einem geeigneten Träger gebildet wird.

10. Mehrschichtiger Film erhältlich durch das Verfahren nach einem oder mehreren der Patentansprüche 1 bis 9.

11. Mehrschichtiger Spinnenseidenproteinfilm, der mindestens zwei Schichten von Spinnenseidenproteinfilmen umfasst, wobei die Filme aus Ameisensäure gegossen sind.

12. Mehrschichtiger Film nach Patentanspruch 11, wobei jede einzelne Schicht des mehrschichtigen Films aus einer Spinnenseidenproteinlösung gebildet ist, die eine oder mehrere Arten von Spinnenseidenprotein umfasst.

13. Mehrschichtiger Film nach Patentansprüchen 11 oder 12, wobei der mehrschichtige Film aus Schichten, die die gleichen (homogener mehrschichtiger Film) oder verschiedene (heterogener mehrschichtiger Film) Spinnenseidenproteine umfassen, gebildet ist, und/oder
wobei der mehrschichtige Film eine oder mehrere aus Spinnenseidenproteinen hergestellte Schichten und eine oder mehrere Schichten, die andere proteinartige oder nicht-proteinartige Materialien umfassen, umfasst,
wobei das nicht-proteinartige Material vorzugsweise aus Polystyrol, Polyvinylchlorid, Poly(styrolsulfonat) (PSS), Poly(allylaminhydrochlorid) (PAH), Poly(acrylsäure) (PAA) und/oder Poly(diallyldimethylammoniumchlorid) (PDADMAC) ausgewählt ist und/oder wobei das andere proteinartige Material vorzugsweise aus Kollagenen, Elastin oder Keratin ausgewählt ist.

14. Mehrschichtiger Film nach einem oder mehreren der Patentansprüche 11 bis 13, wobei die Spinnenseidenproteine natürliche oder rekombinante Seidenproteine sind, vorzugsweise Seiden von Arachnida oder Analoga davon.

15. Mehrschichtiger Film nach einem oder mehreren der Patentansprüche 11 bis 14, wobei die Schichten des mehrschichtigen Films einen oder mehrere Wirkstoffe umfassen, die darin inkorporiert sind oder sich zwischen zwei benachbarten Schichten befinden, wobei der eine oder die mehreren Wirkstoffe vorzugsweise aus Metallen, Chemikalien und/oder pharmazeutischen Wirkstoffen ausgewählt sind und/oder wobei die Spinnenseidenproteine kovalent funktionalisiert sind.

## Revendications

1. Procédé pour former des films multicouche de protéines de soie d'araignée, comprenant les étapes de:
a) fourniture d'une ou plusieurs solutions de protéines de soie d'araignée, dissoutes ou en suspension dans l'acide formique;
b) transformation de l'une desdites solutions en un film;
c) évaporation de l'acide formique, formant ainsi une première couche de film de protéines de soie d'araignée; et
d) une ou plusieurs étapes a) - c) répétitives pour former des couches additionnelles de film de protéines de soie d'araignée sur ladite première couche de film de protéines de soie d'araignée, afin de former un film de protéines de soie d'araignée multicouche.

2. Procédé selon la revendication 1, dans lequel chaque couche simple du film multicouche est formée à partir d'une solution de protéines de soie d'araignée comprenant un ou plusieurs types de protéines de soie d'araignée.

3. Procédé selon l'une ou plusieurs des revendications précédentes, dans lequel le film multicouche est formé à partir de couches comprenant des protéines de soie d'araignée identiques (film multicouche homogène) ou différentes (film multicouche hétérogène).

4. Procédé selon l'une ou plusieurs des revendications précédentes, dans lequel le film multicouche comprend une ou plusieurs couches obtenues à partir de protéines de soie d'araignée et une ou plusieurs couches comprenant d'autres matières protéinacées ou non-protéinacées, tandis que la matière non-protéinacée est de préférence choisie parmi polystyrène, poly(chlorure de vinyle), poly(sulfonate de styrène) (PSS), poly(chlorhydrate d'allylamine) (PAH), poly(acide acrylique) (PAA), et/ou poly(chlorure de diallyldiméthylammonium) (PDADMAC), et/ou tandis que l'autre matière protéinacée est de préférence choisie parmi les collagènes, l'élastine ou la kératine.

5. Procédé selon l'une ou plusieurs des revendications précédentes, dans lequel les protéines de soie d'araignée sont des protéines de soie naturelles ou recombinantes, de préférence de soies provenant d'Arachnides ou leurs analogues.

6. Procédé selon l'une ou plusieurs des revendications précédentes, dans lequel les couches du film multicouche comprennent un ou plusieurs agents qui y sont incorporés ou localisés entre deux couches adjacentes, tandis que le ou les agents sont de préférence choisis parmi les métaux, les composés chimiques et/ou les agents pharmaceutiques.

7. Procédé selon l'une ou plusieurs des revendications précédentes, dans lequel les protéines de soie d'araignée sont fonctionnalisées par covalence avant ou après l'étape 1b).

8. Procédé selon l'une ou plusieurs des revendications précédentes, dans lequel la solution de protéines de soie d'araignée contient 0,1-20%, de préférence 0,5-10%, plus préférablement 1-3% en poids/volume de protéines de soie d'araignée.

9. Procédé selon l'une ou plusieurs des revendications précédentes, dans lequel le film est formé par moulage, revêtement en rotation ou coulée de la solution sur un support approprié.

10. Film multicouche apte à être obtenu par le procédé selon l'une ou plusieurs des revendications 1-9.

11. Film de protéines de soie d'araignée multicouche, comprenant au moins deux couches de films de protéines de soie d'araignée, tandis que lesdits films sont coulés à partir d'acide formique.

12. Film multicouche selon la revendication 11, dans lequel chaque couche simple du film multicouche est formée à partir d'une solution de protéines de soie d'araignée comprenant un ou plusieurs types de protéines de soie d'araignée.

13. Film multicouche selon les revendications 11 ou 12, dans lequel le film multicouche est formé à partir de couches comprenant des protéines de soie d'araignée identiques (film multicouche homogène) ou différentes (film multicouche hétérogène), et/ou
dans lequel le film multicouche comprend une ou plusieurs couches obtenues à partir de protéines de soie d'araignée et une ou plusieurs couches comprenant d'autres matières protéinacées ou non-protéinacées, tandis que la matière non-protéinacée est de préférence choisie parmi polystyrène, poly(chlorure de vinyle), poly(sulfonate de styrène) (PSS), poly(chlorhydrate d'allylamine) (PAH), poly(acide acrylique) (PAA), et/ou poly(chlorure de diallyldiméthylammonium) (PDADMAC), et/ou tandis que l'autre matière protéinacée est de préférence choisie parmi les collagènes,
l'élastine ou la kératine.

14. Film multicouche selon l'une ou plusieurs des revendications 11-13, dans lequel les protéines de soie d'araignée sont des protéines de soie naturelles ou recombinantes, de préférence des soies provenant d'Arachnides ou leurs analogues.

15. Film multicouche selon l'une ou plusieurs des revendications 11-14, dans lequel les couches du film multicouche comprennent un ou plusieurs agents qui y sont incorporés ou localisés entre deux couches adjacentes, tandis que le ou les agents sont de préférence choisis parmi les métaux, les composés chimiques et/ou les agents pharmaceutiques, et/ou tandis que les protéines de soie d'araignée sont fonctionnalisées de manière covalente.
